# EUROPEAN PATENT SPECIFICATION

(11) **EP 1 212 039 B1**
(45) Date of publication and mention of the grant of the patent: **02.11.2005**
(21) Application number: 00957496.3
(22) Date of filing: 15.08.2000
(51) Int. Cl.: A61K 9/00, A61K 31/35

(54) **COMPOSITION FOR INHALATION COMPRISING DELTA-9-TETRAHYDROCANNABINOL IN A SEMIAQUEOUS SOLVENT**
ZUSAMMENSETZUNG FÜR INHALIERUNG ENTHALTEND DELTA-9-TETRAHYDROCANNABINOL IN EINEM SEMI-WÄSSRIGEN LÖSUNGSMITTEL
COMPOSITION POUR INHALATION COMPRENANT DU DELTA-9-TETRAHYDROCANNABINOL DANS UN SOLVANT SEMI-AQUEUX

(30) Priority: 20.08.1999 US 150023 P
(43) Date of publication of application: 12.06.2002
(73) Proprietor: Unimed Pharmaceuticals Inc., Deerfield, IL 60015 (US)
(72) Inventor: MCPHILLIPS, Andrea, M., Columbus, OH 43228 (US); ECONOMOU, Julia, J., Hilliard, OH 43026 (US); DEDHIYA, Mahendra, G., Doublin, OH 43106 (US); WYNNE, Beverly, Ann, Worthington, OH 43085 (US)
(74) Representative: Gosmann, Martin
(86) International application number: PCT/US2000/022495
(87) International publication number: WO 2001/013886

(56) References cited:
- WO-A-00/24362
- L. VACHON, A. ROBBINS, E.A. GAENSLER (S. COHEN AND R.C. STILLMAN, EDS.): "Airways response to aerosolized delta-9-tetrahydrocannabinol: preliminary report" 1976 , THE TERAPEUTIC POTENTIAL OF MARIHUANA. PLENUM MEDICAL BOOK COMPANY , NEW YORK XP000965573 Chapter 8 page 111 -page 121 page 112, paragraph 2 page 113, paragraph 1 - paragraph 2 page 119, paragraph 1 - paragraph 2
- WILLIAMS SJ ET AL: "Bronchodilator effect of delta1-tetrahydrocannabinol administered by aerosol of asthmatic patients" THORAX,GB,LONDON, vol. 31, no. 6, December 1976 (1976-12), pages 720-723, XP002114271
- LICHTMAN ARON H ET AL: "Pharmacological evaluation of aerosolized cannabinoids in mice." EUROPEAN JOURNAL OF PHARMACOLOGY, vol. 399, no. 2-3, 2000, pages 141-149, XP000961027 ISSN: 0014-2999

## Description

### BACKGROUND OF THE INVENTION

### 1. Field of the invention.

The present invention relates to a fast-acting delivery system for delta-9-tetrahydrocannabinol (dronabinol) to improve bioavailability. More particularly, it provides a stable composition for delivery by inhalation to the lungs, and subsequently to the bloodstream, the composition comprising an a therapeutically effective amount of delta-9-tetrahydrocannabinol (also known as "delta-9-THC") and a pharmaceutically-acceptable semiaqueous solvent.

### 2. Statement of the Related Art

Delta-9-tetrahydrocannabinol is currently approved by regulatory authorities for use as an antiemetic in cancer chemotherapy as well as an appetite stimulant for patients inflicted with the AIDS virus. The product is currently marketed under the name MARINOL® (dronabinol) as an oral soft gelatin capsule in which the drug substance is dissolved in sesame oil.

Bioavailability of the current formulation ranges from 10-20% due to a high first pass metabolism associated with oral administration. The current formulation has an onset of action ranging from 0.5 to 1 hour. It would be desirable to improve bioavailabity and quicken onset of action for the above indications as well as for the treatment of alternative conditions, such as spinal cord spasticity, glaucoma, and Alzheimer's disease. Alternative routes previously suggested to overcome oral delivery limitations include the administration of drugs (including delta-9-tetrahydrocannabinol) through the inhalation route. It has been demonstrated in the literature, for example, that smoking marijuana cigarettes (the main constituent being dronanbinol, i.e., delta-9-THC) has shown improved bioavailability (60-70%). However, there are obvious disadvantages relating to smoking marijuana, including raw material impurities, depression of alveolar macrophage activity, and bronchial irritation. Another approach suggested in initial reports at a meeting on February 24, 1998, sponsored by the Institute of Medicine, National Academy of Sciences, Division of Neuroscience and Behavioral Health in Washington, D.C., was to study and use particle size data developed in a conventional nebulizer system to try to enhance bioavailability of delta-9-tetrahydrocannabinol after deep lung route of administration. Among the suggested routes of administration suggested by the prior art are those using aerosol formulations to be inhaled as described in Volicer, U.S. Patent 5,804,592, granted September 8, 1998, based on Provisional Application priority May 7, 1997. However, as presently advised, there has been no prior disclosure of experiments which used formulations comprising delta-9-tetrahydrocannabinol and semiaqueous solvents comprising judiciously selected volumetric ratios of alcohol, water and pharmaceutically-acceptable glycols to enhance partitioning, and no evidence of enhanced bioavailability in warm-blooded animals, including humans, has been known for such compositions prior to the present invention. It still remains desirable, therefore, to develop a new safe, fast acting delivery system for delta-9-tetrahydrocannabinol to improve bioavailability, and such a system is the subject matter of the present invention.

### SUMMARY OF THE INVENTION

In accordance with the present invention there are provided stable compositions for rapid delivery by inhalation to the lungs, and subsequently to the bloodstream, the compositions comprising a therapeutically effective amount of delta-9-tetrahydrocannabinol in a pharmaceutically-acceptable semiaqueous solvent comprising a volumetric ratios of 10-70 parts of an alcohol, 10-30 parts of water and 20-80 parts of glycol, having a combined total of 100, said volumetric ratios of alcohol:water:glycol being sufficient
(i) to aerosolize the composition to a mean mass median aerodynamic diameter in the range of from about 1 up to about 10 µM; and
(ii) to enhance partitioning by producing a stable clear solution near the solubility point of the delta-9-tetrahydrocannabinol.

Among the preferred features of the invention are such compositions wherein
the delta-9-tetrahydrocannabinol comprises from about 0.1 to about 200 mg/mL, and especially 25 and 50 mg/mL;
the solvent comprises ethanol, water and propylene glycol;
the volumetric ratios of ethanol : water : propylene glycol are selected from those in the range of from 10 - 70 : 10 - 30 : 20 - 80, respectively, having a combined total of 100;
the volumetric ratios of ethanol : water : propylene glycol are selected from those in the range of from 10 - 70 : 10 : 20 - 80, respectively, having a combined total of 100;
the volumetric ratios of ethanol : water : propylene glycol are 35 : 10 : 55, respectively, having a combined total of 100.

Also contemplated by the present invention are sterile or preserved sealed unit- and/or multi-unit dosage forms of delta-9-tetrahydrocannabinol comprising a container and a stable composition for rapid delivery by inhalation to the lungs and subsequently to the bloodstream, as first defined above, and especially those wherein the container comprises Type I Amber Glass with a suitable liner.

### DETAILED DESCRIPTION OF THE INVENTION

Numerous experiments have shown that the drug formulation is critical for delta-9-tetrahydrocannabinol to be effectively delivered to the lung rapidly. It has been discovered that the formulations of the present invention must be stable, aerosolize to a particle size less than or equal to 10 µM to reach the lung, and the drug must readily partition out of the delivery system in order to transport across biological membranes and reach the blood stream.

The physico-chemical characteristics of delta-9-tetrahydrocannabinol raw drug material lend themselves to various formulations, including solutions. Delta-9-tetrahydrocannabinol is virtually insoluble in water (0.003 g/mL). It is known that the drug substance is extremely lipophilic, with a reported oil/water coefficient of 9,400,000 (Garret and Hunt, Journal of Pharmaceutical Sciences, Vol. 63, No. 7, pages 1056-1064, 1974; and Thomas et al., The Journal of Pharmacology and Experimental Therapeutics, Vol. 255, No. 1, pages 624-630, 1990). These factors have been considered in developing the compositions of this invention.

Also critical to the present invention is the need for selecting substances which will release the drug for absorption or partition it from the dosage form. The lipophilic nature of delta-9-tetrahydrocannabinol suggests that formulations made primarily of lipophilic excipients such as oils, such as sesame seed oil, currently approved for oral unit dosage use, would not be desirable because the drug would not partition readily. In the case of oily excipients, delta-9-tetrahydrocannabinol would have a strong affinity for the formulation and would slowly partition out, resulting in slow absorption, exactly the problem sought to be avoided.

Semiaqueous solutions, that is combinations of organic solvents with small, effective amounts of water, lend themselves to making formulations with delta-9-tetrahydrocannabinol with unexpected increases in partitioning, apparently because the drug has a poor affinity for the water within the formulation. Because of the increased ease of partitioning, once released deeply in the lung from the dosage forms of the present invention, delta-9-tetrahydrocannabinol is able to cross cell membranes rapidly, traverse the alveolar epithelial cells, interstitium, and endothelium to reach the blood stream (Thompson, "Pharmacology of Therapeutic Aerosols" Chapter 2, in Pharmaceutical Inhalation Aerosol Technology, Ed. Hickey, Marcel Dekker, Inc. New York, pages 29-37, 1992). As a further advantage, the formulations of delta-9-THC and semiaqueous solvents of the present invention may be aerosolized more easily than oil based systems.

As will be shown hereinafter, delta-9-tetrahydrocannabinol readily dissolves in ethanol and in equal parts of ethanol and propylene glycol to form clear solutions which, for purposes of the present invention, are "stable" that is, remain clear through three cycles of freeze/thaw. Such compositions, however, do not meet the ease of partitioning required by the present invention because the delta-9-tetrahydrocannabinol prefers to stay in the organic phase and only slowly releases itself from the dosage form at the intended site of absorption. As will also be described in detail hereinafter, water can be added to the organic phase, and the delta-9-tetrahydrocannabinol is able to remain in solution, near the solubility point of the drug, and, unexpectedly, partitioning is enhanced and in vivo bioavailability is accelerated, especially in comparison with i.v. administration of the same formulation. The experiments have also shown that as the water content of the semiaqueous solvent increases and the ethanol content decreases beyond a certain level, the drug readily falls out of solution, and such unstable formulations no longer function as dosage forms within the scope of the invention.

The citation to Thomas, mentioned above, teaches that aerosol particle size has an influence on the deposition pattern of many drugs in the lung. In general, deposition is successful at a mean mass median aerodynamic diameter in the range of from about 1 µM to about 10 µM. For best results in lung delivery, it is known from Thomas that delta-9-tetrahydrocannabinol should be targeted for delivery deep in the lung, and this is facilitated by using aerosol particle diameters of less than about 3 µM, a size which is readily, but unexpectedly, obtained with the compositions of the present invention, using conventional nebulizers, as will be shown in the examples which follow, and in conventional metered dose inhalers.

To make the formulations of the present invention, it is preferred, but not essential, to dissolve, per mL of final composition, 1-250 mg of delta-9-tetrahydrocannabinol (dronabinol), USP in 5-95% v/v of ethanol, USP (190 proof), or an obvious equivalent, e.g., isopropanol, in a suitable mixer; to add 20-80% v/v propylene glycol, USP, or an obvious equivalent, such as polypropylene glycol, polyethylene glycol, and the like, and 10-25% v/v purified water and mix, and then filter and transfer to a storage tank. A suitable concentration of delta-9-tetrahydrocannabinol in pharmaceutical compositions for inhalation is 0.05 to 15% (by weight). A preferred concentration is from 0.02 to 5%. A more preferred concentration is from 0.1 to 4%. The formulations of the invention can also include minor but effective amounts of anti-oxidants, surfactants, buffers, sodium chloride, pH adjusting agents, bacteriostats, stabilizers, preservatives, and the like.

To package the formulations of the present invention, the formulations are transferred by conventional means to unit-dose or multi-dose sealed containers, such as ampules and vials, preferably made of amber glass Types I, II and III, more preferably Type I, with a suitable liner.

To use the formulations of the present invention, the quantity of delta-9-tetrahydrocannabinol can vary widely. For example, the amount may be from about 0.001 to 35 mg/kg of body weight administered one to six times per day. However, the dose administered to an animal, particularly a human, should be sufficient to effect a therapeutic response over a reasonable time frame. The dose will be determined by the strength of the particular compositions employed and the condition of the person, as well as the body weight of the person to be treated. The size of the dose also will be determined by the existence, nature and extent of any adverse side-effects that might accompany the administration of a particular composition. A suitable dosage of delta-9-tetrahydrocannabinol for administration by inhalation is 0.01 to 100 mg/kg per day, given in 2-4 divided doses. A preferred dosage is 0.01 to 35 mg/kg per day. A more preferred dosage is 0.05 to 5 mg/kg per day.

### DESCRIPTION OF THE PREFERRED EMBODIMENTS

The following examples illustrate the present invention. They are not to be construed to limit the claims in any manner whatsoever. Semiaqueous solvent ratios are volumetric, i.e., v/v, and total 100 parts.

### EXAMPLES 1-7

The physical stability of delta-9-tetrahydrocannabinol in varying ratios of ethanol, USP, purified water and propylene glycol is determined by placing 0.3 ml of delta-9-tetrahydrocannabinol (Standard, 100 mg/mL) in a 16x100 mm Pyrex tube, adding 2.7 mL of absolute ethanol for a total volume of 3.0 mL, and shaking the tube to mix. This results in a 100:0:0 ethanol (E); water (W): propylene glycol (PG) ratio and a drug concentration of 10 mg/mL. The foregoing steps are repeated for 12 more ratios according to the following Table 1, which lists volumes of standard delta-9-tetrahydrocannabinol (delta-9-THC), ethanol (Alcohol), purified water (Water), and propylene glycol, and the visual inspection results recorded after the samples are placed on a freeze/thaw (F/T) cycle for three turns.

**Table 1.**

| Delta-9-THC in Solvent Systems | | |
|---|---|---|
| Example | Ratio (v/v) (Alcohol:Water: Polypropylene Glycol) | Visual Observation after 3 F/T cycles |
| 1A* | 100:0:0 | Clear |
| 1B* | 50:0:50 | Clear |
| 1 | 70:10:20 | Clear |
| 2 | 60:10:30 | Clear |
| 3 | 50:10:40 | Clear |
| 4 | 40:10:50 | Clear |
| 5 | 30:10:60 | Clear |
| 5* | 10:10:80 | Clear/oil droplets form when shaken |
| 6 | 60:20:20 | Clear |
| 7 | 40:20:40 | Clear |
| 7A* | 20:20:60 | Clear/oil droplets form when shaken |
| 7B* | 30:25:45 | Cloudy/oil droplets visible |
| 7C* | 35:30:35 | Cloudy |

| | | |
|---|---|---|
| * - Comparison - 1A and 1B are not semiaqueous solvents and 5A, 7A, 7B and 7C are not stable after freeze/thaw. | | |

As can be seen from Table 1, in varying ratios of ethanol/propylene glycol, delta-9-tetrahydrocannabinol is able to remain in solution in the presence of controlled amounts of water. However, as the water content increases and ethanol content decreases beyond a certain level, the drug readily falls out of solution.

### EXAMPLES 8-14

The procedure of Examples 1-7 is repeated to assess solubility of increasing concentration of delta-9-tetrahydrocannabinol in a selected vehicle. Based on freeze/thaw data generated with delta-9-tetrahydrocannabinol using different solvent ratios (Table 1) a vehicle comprised of alcohol/water/propylene glycol in a volumetric ratio of 35:10:55 is selected. This ratio allows for good solubility of the drug while keeping the alcohol concentration low enough for ease of manufacturing. Results of the experiments are set forth in Table 2:

**Table 2.**

| Solubility of Delta-9-tetrahydrocannabinol in Alcohol :Water:Propylene Glycol (35:10:55) (v/v) | | |
|---|---|---|
| Example | Delta-9-THC Conc. | Visual Observation |
| 8 | 0.16mg/mL¹ | Clear, colorless soln. |
| 9 | 0.40mg/mL¹ | Clear, colorless soln. |
| 10 | 0.80mg/mL¹ | Clear, colorless soln. |
| 11 | 25 mg/mL | Clear, light yellow soln. |
| 12 | 50 mg/mL | Clear, light yellow soln. |
| 13 | 75 mg/mL | Clear, light yellow soln. |
| 14 | 100 mg/mL | Clear, light yellow soln. |
| 14A* | 200 mg/mL | Cloudy, yellow soln. |

| | | |
|---|---|---|
| ¹ - Prepared by sequential dilution of Example 11 | | |
| * - Comparative Example (fails to enhance partionability) | | |

The results show that if the alcohol concentration is reduced below approximately 35%, drug droplets begin to form indicating drug is below its solubility point in the vehicle. The results also indicate that delta-9-tetrahydrocannabinol concentrations in excess of 100 mg/mL are able to be manufactured with this formulation, but 200 mg/mL cannot.

From ease of manufacturing and expected doses of delta-9-tetrahydrocannabinol required for inhalation, a drug concentration of 25 mg/mL in the formulation of Example 8 (35:10:55 Alcohol:Water:Propylene Glycol) is evaluated in preclinical studies. A Pari LC Plus Nebulizer is used in a conventional fashion and generates aerosolized particles having a mean mass median aerodynamic diameter of 2.96 µM, which is well within the desired particle size for deep lung delivery.

Two animal species, rat and dog, are administered the formulation in a 14 day multiple dose inhalation study with a nebulizer. Results from the pharmacokinetic portion of the study indicate comparability between tₘₐₓ values found for intravenous and inhaled delivery. Tₘₐₓ values are summarized for both single (intravenous and inhalation) and for multiple dose (intraveneous and inhalation) of delta-9-tetrahydrocannabinol in Table 3, as follows:

**Table 3.**

| Animal Studies of Exposure to Inhaled Delta-9-Tetrahydrocannabinol in Alcohol :Water:Propylene Glycol (35:10:55)(v/v) | | | | | |
|---|---|---|---|---|---|
| Single Dose Administration | | | | | |
| Species | Route of Admin. | Dose | Duration of Exposure | Average tₘₐₓ (minutes) | App.Calc.tₘₐₓ (minutes)¹ |
| Dog | IV | 1 mg/kg | --- | 1.8 | 1.8 |
| | Inhalation | 2 " | 8 minutes | 15.6 | 7.6 |
| Rat | IV | 2 mg/kg | --- | 2.1 | 2.1 |
| | Inhalation | 0.5 " | 1.25 minutes | 5.7 | 4.45 |
| | Inhalation | 4.8 " | 15 minutes | 33.3 | 18.3 |

| Multiple Dose Inhalation | | | | | |
|---|---|---|---|---|---|
| Species | Route of Admin.² | Dose | Duration of Exposure | Average tₘₐₓ (minutes) | App.Calc.tₘₐₓ (minutes)¹ |
| Dog | IV | 1 mg/kg | --- | 1.8 | 1.8 |
| | Inhalation | 2 mg/kg | 8 minutes | 10.2 | 2.2 |
| | Inhalation | 5 " | 20 minutes | 22.2 | 2.2 |
| | Inhalation | 15 " | 60 minutes | 66 | 6 |
| Rat | IV | 2 mg/kg | --- | 2.1 | 2.1 |
| | Inhalation | 2 mg/kg | 5 minutes | 6 | 1 |
| | Inhalation | 5 " | 15 minutes | 17.4 | 2.4 |
| | Inhalation | 15 " | 45 minutes | 46.2 | 1.2 |

| | | | | | |
|---|---|---|---|---|---|
| ¹Apparent tₘₐₓ calculated as (tₘₐₓ-duration of exposure). Blood samples drawn after entire dose is administered. | | | | | |
| ²IV data represents single dose administration. | | | | | |

The data in the foregoing examples show that a semiaqueous formulation of delta-9-tetrahydrocannabinol in accordance with the present invention can produce a stable clear solution near the solubility point of the drug. Moreover, because delta-9-tetrahydrocannabinol has poor affinity for the formulation, it is able to partition out and transport across cell membranes to reach the bloodstream quickly. This has been demonstrated by the comparative tₘₐₓ values achieved in single dose intravenous and 14 day multiple dose inhalation studies conducted in dogs and rats.

For example as a raw drug, synthetic or natural source-derived delta-9-tetrahydrocannabinol can be used, as well as prodrugs, isomers, derivatives, metabolites, and the like. Generally a wide variety of hydroxy containing solvents can be used, such as isopropanol instead of ethanol and polypropylene glycol instead of propylene glycol, so long as they are pharmaceutically-acceptable.

## Claims

1. A stable composition for rapid delivery by inhalation to the lungs, and subsequently to the bloodstream, said composition comprising a therapeutically effective amount of delta-9-tetrahydrocannabinol in a pharmaceutically-acceptable semiaqueous solvent comprising volumetric ratios of 10 - 70 parts of an alcohol, 10 - 30 parts of water and 20 - 80 parts of a glycol, having a combined total of 100, said volumetric ratios of alcohol : water : glycol being sufficient
(i) to aerosolize the composition to a mean mass median aerodynamic diameter in the range of from about 1 up to about 10 µM; and
(ii) to enhance partitioning by producing a stable clear solution near the solubility point of the delta-9-tetrahydrocannabinol.

2. A composition as defined in Claim 1 wherein said delta-9-tetrahydrocannabinol comprises from 0.1 to 200 mg/mL.

3. A composition as defined in Claim 2 wherein said delta-9-tetrahydrocannabinol comprises from 0.1 to 25 mg/mL.

4. A composition as defined in Claim 2 wherein said delta-9-tetrahydrocannabinol comprises 50 mg/mL.

5. A composition as defined in Claim 1 wherein said solvent comprises ethanol, water and propylene glycol.

6. A composition as defined in Claim 5 wherein the volumetric ratios of ethanol : water: propylene glycol are selected from those in the range of from 10 - 70 : 10 - 30 : 20 - 80, respectively, having a combined total of 100.

7. A composition as defined in Claim 6 wherein the volumetric ratios of ethanol : water: propylene glycol are selected from those in the range of from 10 - 70 : 10 : 20 - 80, respectively, having a combined total of 100.

8. A composition as defined in Claim 7 wherein the volumetric ratios of ethanol : water: propylene glycol are 35 : 10 : 55, respectively, having a combined total of 100.

9. A sterile and/or preserved sealed unit- or multi-unit dosage form of delta-9-tetrahydrocannabinol comprising a container and a stable composition for rapid delivery by inhalation to the lungs and subsequently to the bloodstream, as defined in Claim 1.

10. A sterile and/or preserved sealed unit- or multi-unit dosage form as defined in Claim 9 wherein said container comprises Type I Amber Glass with a suitable liner.

## Patentansprüche

1. Stabile Inhalationszusammensetzung für eine rasche Abgabe an die Lungen und anschließend an den Blutstrom, welche Zusammensetzung eine therapeutisch wirksame Menge an Delta-9-Tetrahydrocannabinol in einem pharmazeutisch annehmbaren halbwässerigen Lösungsmittel umfaßt, das Volumenverhältnisse von 10 - 70 Teilen eines Alkohols, 10 - 30 Teilen Wasser und 20 bis 80 Teilen eines Glykols, die zusammen einen Gesamtwert von 100 ergeben, aufweist, welche Volumenverhältnisse von Alkohol:Wasser:Glykol dazu ausreichen,
(i) die Zusammensetzung in ein Aerosol mit einem mittleren massebezogenen aerodynamischen Median-Durchmesser im Bereich von etwa 1 bis zu etwa 10 µm überzuführen; und
(ii) die Verteilung durch Ausbilden einer stabilen klaren Lösung nahe dem Löslichkeitspunkt des Delta-9-Tetrahydrocannabinols zu fördern.

2. Zusammensetzung nach Anspruch 1, worin das Delta-9-Tetrahydrocannabinol von 0,1 bis 200 mg/ml ausmacht.

3. Zusammensetzung, nach Anspruch 2, worin das Delta-9-Tetrahydrocannabinol von 0,1 bis 25 mg/ml ausmacht.

4. Zusammensetzung nach Anspruch 2 , worin das Delta-9-Tetrahydrocannabinol 50 mg/ml ausmacht.

5. Zusammensetzung nach Anspruch 1, worin das Lösungsmittel Ethanol, Wasser und Propylenglykol umfaßt.

6. Zusammensetzung nach Anspruch 5, worin die Volumenverhältnisse von Ethanol:Wasser:Propylenglykol aus jenen im Bereich von 10-70:10-30:20-80 ausgewählt sind, die zusammen einen Gesamtwert von 100 ergeben.

7. Zusammensetzung nach Anspruch 6, worin die Volumenverhältnisse von Ethanol:Wasser:Propylenglykol aus jenen im Bereich von 10-70:10:20-80 ausgewählt sind, die zusammen einen Gesamtwert von 100 ergeben.

8. Zusammensetzung nach Anspruch 7, worin die Volumenverhältnisse von Ethanol:Wasser:Propylenglykol 35:10:55, die zusammen einen Gesamtwert von 100 ergeben, betragen.

9. Sterile und/oder konservierte Dosierungsform von Delta-9-Tetrahydrocannabinol in einer versiegelten Einheit oder Mehrfacheinheit, umfassend einen Behälter und eine stabile Inhalationszusammensetzung für eine rasche Abgabe an die Lungen und anschließend an den Blutstrom, wie in Anspruch 1 definiert.

10. Sterile und/oder konservierte Dosierungsform in verschlossener Einheit oder Mehrfacheinheit, wie in Anspruch 9 definiert, worin der Behälter ein Typ I-Gelbglas mit einem geeigneten Liner umfaßt.

## Revendications

1. Composition stable pour une délivrance rapide par inhalation aux poumons, et par la suite au courant sanguin, ladite composition comprenant une quantité thérapeutiquement efficace de delta-9-tétrahydrocannabinol dans un solvant semi-aqueux pharmaceutiquement acceptable comprenant des rapports volumétriques de 10 à 70 parties d'un alcool, 10 à 30 parties d'eau et 20 à 80 parties d'un glycol, présentant un total combiné de 100, lesdits rapports volumétriques alcool:eau:glycol étant suffisants
(i) pour transformer la composition en aérosol à un diamètre aérodynamique médian en masse moyenne situé dans l'intervalle d'environ 1 à environ 10 µM ; et
(ii) pour amplifier la division en produisant une solution limpide stable proche du point de solubilité du delta-9-tétrahydrocannabinol.

2. Composition selon la revendication 1, dans laquelle ledit delta-9-tétrahydrocannabinol est présent à raison de 0,1 à 200 mg/ml.

3. Composition selon la revendication 2, dans laquelle ledit delta-9-tétrahydrocannabinol est présent à raison de 0,1 à 25 mg/ml.

4. Composition selon la revendication 2, dans laquelle ledit delta-9-tétrahydrocannabinol est présent à raison de 50 mg/ml.

5. Composition selon la revendication 1, dans laquelle ledit solvant comprend de l'éthanol, de l'eau et du propylèneglycol.

6. Composition selon la revendication 5, dans laquelle les rapports volumétriques éthanol:eau:propylèneglycol sont choisis parmi ceux situés dans l'intervalle de 10 - 70:10 - 30:20 - 80, respectivement, présentant un total combiné de 100.

7. Composition selon la revendication 6, dans laquelle les rapports volumétriques éthanol:eau:propylèneglycol sont choisis parmi ceux situés dans l'intervalle de 10 - 70:10:20 - 80, respectivement, présentant un total combiné de 100.

8. Composition selon la revendication 7, dans laquelle les rapports volumétriques éthanol:eau:propylèneglycol sont de 35:10:55, respectivement, présentant un total combiné de 100.

9. Forme posologique unitaire ou multi-unitaire fermée hermétiquement, stérile et/ou conservée, de delta-9-tétrahydrocannabinol, comprenant un récipient et une composition stable pour la délivrance rapide par inhalation aux poumons, et par la suite au courant sanguin selon la revendication 1.

10. Forme posologique unitaire ou multi-unitaire fermée hermétiquement, stérile et/ou conservée, selon la revendication 9, dans laquelle ledit récipient est constitué de verre Ambre de type I avec une chemise appropriée.
